# EUROPEAN PATENT APPLICATION

(11) **EP 0 559 411 A1**
(43) Date of publication of application: **08.09.1993**
(21) Application number: 93301526.5
(22) Date of filing: 01.03.1993
(51) Int. Cl.: A61M 35/00

(54) **Titratable transdermal patch system and method for administration of therapeutic substances**

(30) Priority: 04.03.1992 US 846519
(71) Applicant: ELI LILLY AND COMPANY, Indianapolis, Indiana 46285 (US)
(72) Inventor: Valia, Kirti Himatlal, Plainsboro, New Jersey 08536 (US); Wernicke, Joachim Franz, League City, Texas 77573 (US)
(74) Representative: Tapping, Kenneth George

(57) **Abstract**

A system for providing variable levels of transdermal administration of a therapeutic substance is disclosed which comprises a sheet of interconnected bandage elements (12) defined by a plurality of separation lines (13), such as perforation lines. Each bandage element includes a sandwich-type laminate including a backing lamina (14) and contact adhesive lamina (15) connected thereto. Each bandage element further includes a predetermined amount of a releasable form of a therapeutic substance which communicates to the skin by way of the adhesive lamina. A desired number of bandage elements are removed from the sheet along the separation lines and are then applied to the skin. The dosage of the therapeutic substance is readily titrated by varying the number of bandage elements used.

## Description

### BACKGROUND OF THE INVENTION

### Field of the Invention:

The present invention relates to a system and method for administering therapeutic substances transdermally, and more particularly to providing variable levels of transdermal administration of therapeutic substances.

### Description of the prior Art:

The administration of systemic drugs and other therapeutic substances transdermally is well known in the art. A variety of commercial products are available for this purpose. In addition, prior art literature has described particular configurations for bandages, commonly referred to as "patches", which are designed to release substances into the skin when adhered thereto.

One of the difficulties in administering therapeutic substances is that the rate of administration must be matched to the individual. Desired dosage rates will differ among individuals, and in some instances it is preferable to vary the rate of administration for a single individual. The prior art has dealt with this problem by providing transdermal bandages having varying sizes or different administration rates of the therapeutic substances. However, this is inconvenient for the manufacturers, hospitals, pharmacies and ultimate users of the bandages.

The present invention has overcome this problem by providing an easily and inexpensively produced bandage system that permits varying the level of transdermal administration of therapeutic substances. The method of using this bandage system is simple and reliable, and avoids the need to supply an assortment of bandage sizes or administration characteristics.

### SUMMARY OF THE INVENTION

Briefly describing one aspect of the present invention, there is provided a system for the administration of a therapeutic substance transdermally which comprises a sheet of interconnected bandage elements defined by a plurality of separation lines. Each bandage element includes a sandwich-type laminate including a backing lamina, and a contact adhesive lamina connected thereto. Each bandage element further includes a predetermined amount of a releasable form of a therapeutic substance, which is communicatable to the skin by way of the adhesive lamina. In use, a desired number of bandage elements are removed from the sheet along the separation lines, and are then applied to the skin. The number of bandage elements selected determines the level of administration of the therapeutic substance. A method for varying the level of transdermal administration of a therapeutic substance is also provided.

It is an object of the present invention to provide a system for transdermal administration of a therapeutic substance, and more particularly to permit selective variation of the level of administration.

A further object of the present invention is to provide a method for variable-rate, transdermal administration of a therapeutic substance which is simple and reliable in use.

Another object of the present invention is to provide systems and methods for administering therapeutic substances transdermally which permit variation of the level of administration without the need to provide a myriad of bandage sizes and administration characteristics.

Further objects and advantages of the present invention will be apparent from the description of the preferred embodiment which follows.

### BRIEF DESCRIPTION OF THE DRAWINGS

FIG. 1 is a perspective view of a transdermal bandage sheet constructed in accordance with the present invention.

FIG. 2 is a cross-sectional view of a typical transdermal bandage useful with the present invention.

FIG. 3 is a cross-sectional view of an alternative type of transdermal bandage useful with the present invention.

### DESCRIPTION OF THE PREFERRED EMBODIMENT

For the purposes of promoting an understanding of the principles of the invention, reference will now be made to the embodiment illustrated in the drawings and specific language will be used to describe the same. It will nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

The present invention provides a system and method for administration of a therapeutic substance transdermally. The invention affords a ready way to vary the rate of administration of the therapeutic substance without providing several different types of transdermal bandages. The bandages used by the present invention are readily fabricated and easily used.

It may be desirable for a variety of reasons to adjust the rate of transdermal administration of a therapeutic substance. Persons will preferably receive dosage rates that are tailored to the situation, which may differ due to size, age or condition of the person, treatment considerations, etc. Also, it may be appropriate to periodically vary the dosage rate for an individual, perhaps due to the nature of the drug, or in response to changes in the person's condition. In another instance, the dosage of certain drugs may be gradually increased, for example to allow the person's body to adjust to the medication or to avoid adverse reactions which would occur if the full strength of the drug was administered immediately.

The present invention permits all of the foregoing desires to be satisfied by providing a transdermal bandage system and method which allows the ready adjustment of the rate of administration of a therapeutic substance. Referring to the drawings, there is shown an exemplary system 10 for transdermal administration. The system 10 comprises a sheet 11 of interconnected bandage elements 12 defined by a plurality of separation lines 13. As further described hereafter, each bandage element may be readily separated from the sheet 11 along the separation lines.

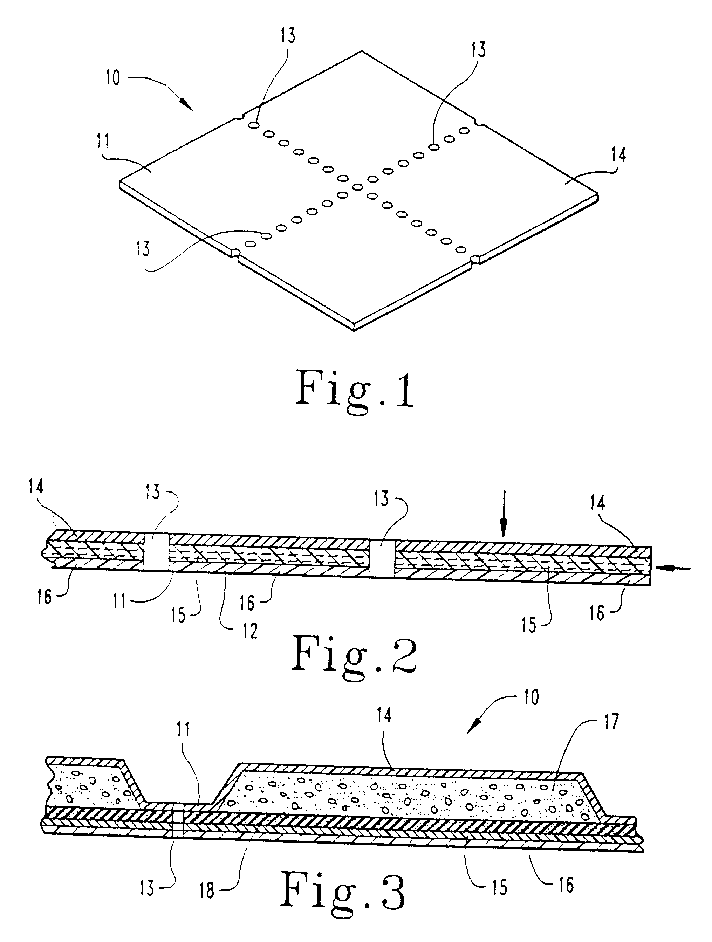

The bandage elements comprise sandwich-type laminates which may have any of a wide variety of configurations. Such laminates are well known in the art, and typically include at least a backing lamina, an adhesive lamina, and a source of a releasable form of the therapeutic substance. The adhesive layer secures the bandage to the skin, and the therapeutic substance is released into the person through the contacted skin. As shown by way of example in FIG. 2, the bandage elements include a backing lamina 14. This backing lamina is preferably an impermeable layer having sufficient strength and durability for use as the support substrate for the bandage. The impermeable backing also keeps any volatile components from escaping. The backing layer may itself comprise a laminate of films of polymer and/or metal foil, such as aluminum foil. Polymers that may be used include high and low density polyethylene, polypropylene, polyvinylchloride and polyethylene terephthalate. As will be appreciated, suitable backing lamina are well known, and their selection are not critical to the present invention. In a preferred embodiment, the backing comprises a polyester film having a thickness of 50 microns, such as that available commercially under the trademark "SCOTCHPAK 1012".

The laminate further includes an adhesive lamina 15 connected, directly or indirectly, with the backing lamina. The adhesive lamina is provided to adhere the bandage element to the skin, and therefore most preferably includes a a pressure-sensitive or "contact" adhesive suitable for securement to the skin. The adhesive must be biologically acceptable, e.g. hypoallergenic, and compatible with the other components of the bandage.

A number of such adhesive materials are known in the art, and the present invention is not limited by the particular adhesive used. Typical adhesives useful in a transdermal patch include polydimethylsiloxane and certain polyacrylic adhesive polymers (in the form of an alkyl ester, amide, free acid, or the like) and polyisobutylene adhesive polymers.

By way of further example, the adhesive lamina may comprise an acrylic polymer comprising a hydrophobic monomeric acrylic or methacrylic acid ester of an alkyl alcohol, and optionally also including a reinforcing monomer, as described in U.S. Patent No. 4,751,087, issued to Wick and hereby incorporated by reference.

Another example of a suitable adhesive is a gelled mixture of mineral oil of about 10 to about 100 cp at 25°C and a blend of polyisobutene, as described in U.S. Patent No. 4,031,894, issued to Urquhart et al. on June 28, 1977 and hereby incorporated by reference. As described therein, such mixtures typically include 35% to 65% of the mineral oil, and the polyisobutene may comprise 10% to 40% of a low molecular weight polyisobutene (35,000-50,000 viscosity average molecular weight) and 20% to 40% of a high molecular weight (1,000,000-1,500,000 viscosity average molecular weight) polyisobutene.

A release liner 16 is initially secured to the adhesive lamina in conventional fashion, and is readily removed prior to use of the bandage. The choice of release liner is not related to the present invention, and is well within the skill in the art. The liner is preferably formed from an impermeable material, such as those from which the backing lamina may be made, with the provision that the liner is made strippable, such as by siliconizing. In a preferred embodiment, this liner 16 comprises a polyester film having a thickness of 75 microns, such as that available commercially under the trademark "SCOTCHPAK 1022".

Also provided in each bandage element is a predetermined amount of a releasable form of the therapeutic substance to be administered transdermally. The substance is releasable in that it will pass from the bandage and to the skin by virtue of the adherence of the bandage to the skin. The therapeutic substance may be included in the bandage element in a variety of ways known in the art, the object being that the substance communicates to the skin by way of the adhesive lamina. For example, the substance may be mixed with the adhesive in the adhesive lamina, or it may be positioned between the adhesive lamina and the backing lamina.

The therapeutic substance is provided in a manner to permit the substance to pass through the outer surface of the adhesive lamina in contact with the user's skin. In the embodiment shown in FIG. 2, the therapeutic substance is combined within the adhesive lamina 15. Upon removal of the release liner 16, the bandage element may be applied to the skin and the therapeutic substance will migrate from the adhesive lamina into the skin. An alternate form of bandage element is shown in FIG. 3, in which at least a portion of the therapeutic substance is provided in a reservoir 17 located between the backing lamina 14, a microporous polymeric membrane 18, and the adhesive lamina 15. In this configuration, the substance in the reservoir migrates through the rate-controlling membrane and the adhesive lamina in order to enter the skin.

The present invention is also useful with a variety of other configurations of bandage laminates. For example, the laminate may include multiple layers having varying concentrations and/or forms of the therapeutic substance. Layers may also be included which have different permeabilities for the substance, thereby regulating the rate of transmission of the substance to the skin. Examples of other known transdermal bandage laminates are contained in U.S. Patent Nos. 4,031,894, issued to Urquhart et al.; 4,797,284, issued to Loper et al.; 4,781,924, issued to Lee et al.; 4,764,381, issued to Bodor et al.; 4,764,379, issued to Sanders et al.; and, 4,814,168, issued to Sablotsky et al. The pertinent portions of the foregoing patents are hereby incorporated by reference.

It is contemplated that each bandage element in a sheet will have a predetermined amount of therapeutic substance, and will have associated with it a predetermined rate of administration of the substance when the element is attached to the skin. It is preferred that the amount and rate of administration of the therapeutic substance be the same for each bandage element. It is further preferred that the therapeutic substance be uniformly applied throughout the area of each bandage element.

The individual bandage elements 12 are interconnected in the form of the sheet 11. The separation lines define a grid which divides the sheet into the several bandage elements. As used herein, the term "separation lines" refers to lines along which the sheet is readily divided into one or more bandage elements. The separation lines can be arranged in any fashion which yields a continuous sheet of interconnected bandage elements. A preferred sheet, for example, includes a grid of perforation lines which divide the sheet into a square or rectangular array of bandage elements, in configuration similar to a sheet of postage stamps, as shown in FIG. 1.

The separation lines may be provided in any manner which facilitates the separation of the sheet. The lines 13 preferably comprise lines of perforations. Alternatively, the lines could, for example, comprise score lines. The release liner 16 is provided with separation lines which conform to those of the bandage elements to facilitate division of the sheet for use.

As shown in the drawings, the separation lines are arranged to permit any desired number of bandage elements to be removed as a group from the sheet 11. The sheet in FIG. 1, for example, could be readily separated into halves. Alternatively, three bandage elements could be separated from the fourth and used as a group. Of course, sheets having larger numbers of bandage elements could be as readily prepared. As a further example, the bandage elements could be provided as a continuous roll having one, two or more separable elements across, and divisible at longitudinal spacings, similar in layout to a roll of postage stamps.

It is preferred that the therapeutic substance be applied evenly over the entire area of the sheet 11. For typical uses, it is also preferred that the separation lines divide the sheet into bandage elements of equal size.

It will be appreciated that the present invention is useful with essentially any therapeutic substance suitable for transdermal administration. The term "therapeutic substance" is used herein in its broad sense as meaning any agent which may be administered transdermally for the purpose of providing some beneficial or therapeutic effect either locally or systemically. In general, the therapeutic substances useful with the present invention include agents in all of the major therapeutic classes including, but not limited to, anti-infectives such as antibiotics and antiviral agents, analgesics and analgesic combinations, anorexics, anthelmintics, antiarthritics, antiasthma agents, anticonvulsants, antidepressants, antidiabetic agents, antidiarrheals, antihistamines, anti-inflammatory agents, antimigraine preparations, antimotion sickness agents, antinauseants, antineoplastics, antiparkinsonism agents, antipruritics, antipsychotics, antipyretics, antispasmodics including gastrointestinal and urinary; dopamine agonists, anticholinergics, sympathomimetics, xanthine derivatives, various cardiovascular agents including calcium channel antagonists, beta-blockers, antiarrhythmics, positive inotropic agents, antihypertensives, diuretics, vasodilators including general, coronary, peripheral and cerebral; central nervous system stimulants, corticosteroids, cough and cold preparations, decongestants, diagnostics, hormones, hypnotics, immunosuppressives, muscle relaxants, parasympatholytics, parasympathomimetics, psychostimulants, sedatives, tranquilizers and the like.

Further specific examples of therapeutic substances useful with the present invention include those listed hereafter: (+)-1,4-dihydro-2-6-dimethyl-4-(3-nitrophenyl)-3,5-pyridinedicarboxylic acid, methyl 1-(phenylmethyl)-3-pyrrolidinyl ester, the free base of a dihydropyridine calcium channel antagonist which is a potent coronary and peripheral vasodilator; 1,3-dihydro-3,3-dimethyl-5-(1,4,5,6-tetrahydro-6-oxo-3-pyridazinyl)-2H-indol-2-one, which possesses both positive inotropic and vasodilator activities; pinacidil (i.e., (±)-N-cyano-N'-4-pyridinyl-N''-(1,2,2-trimethylpropyl)guanidine, monohydrate, an antihypertensive agent; quinelorane (i.e., trans(-)5,5A,6,7,8,9,9A,10-octahydro-6-propylpyrimidino(4,5-G)quinolin-2-amine), the free base of a dopamine agonist; pergolide, a substituted ergoline disclosed in U.S. Patent No. 4,166,182 and proven to be effective in the treatment of some symptoms of parkinsonism; and dobutamine, discussed in EP-A-492930.

Examples of other drugs commonly administered transdermally and referenced in recent patents include nitroglycerin, steroidal pharmaceuticals such as estradiol, nicotine and verapamil. See U.S. Patent Nos. 4,715,087 issued to Wick, 4,615,699 issued to Gale et al., 4,883,669 issued to Chien et al., 4,908,213 issued to Govil et al. and 4,690,683 issued to Chien et al.

The present invention is not restricted as to the type of substance employed. The backing lamina, adhesive lamina and release liner, as well as the overall configuration of the bandage laminate, may simply be selected to be compatible with the substance to be employed. The size of the individual bandage elements is conveniently selected to suit the type, concentration and rate of administration of the therapeutic substance, based on the laminate configuration.

The rate of administration of a therapeutic substance can be controlled in terms of the rate per surface area (e.g., mg/hr/cm²) of the bandage element. The size of the bandage elements may also be adjusted in view of this factor. Thus, the administration rate per surface area and the size of individual bandage elements can be selected in relation to the nature of the therapeutic substance and the configuration of the bandage laminate.

The present invention has particular application for certain drugs which commonly require adjustment or variation of their dosage levels for an individual. For example, dopamine agonists such as pergolide mesylate can produce nausea and vomiting if administered orally at high levels. Currently, pergolide is given orally with a definite titration schedule which takes several weeks to complete, and at the end nausea and other side effects may persist.

This result can be minimized or eliminated by gradually increasing the rate of administration of these drugs, perhaps over a period of days or weeks. During the transdermal administration, the input rate is low and the tachyphylaxis or tolerance develop in several days, with the transdermal approach providing steady-state plasma levels. The titratable patch of the present invention provides a better drug therapy for such dopamine agonists, as well as other drugs.

In accordance with the present invention, the rate of administration can be gradually increased by using successively greater numbers of bandage elements. Using a single sheet, the user first removes and applies a single bandage element. In successive applications the user removes and applies two, three, etc. bandage elements until the desired number, and therefore rate of administration, is reached. If the drug is not effective, then the number of bandage elements is increased. If undesirable side effects occur, then the number of bandage elements is decreased.

The bandage sheets of the present invention are readily fabricated. In one approach, the adhesive layer and other lamina are applied in order onto the backing lamina, and a release liner is applied thereto. The separation lines, such as perforation or score lines, are then made in the sheet, including both the backing lamina and the release liner, in conventional fashion. Alternatively, the backing lamina and release liner are first provided with the separation lines prior to forming the laminate. Other approaches will also be apparent as being within the skill in the art.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

## Claims

1. A system for the administration of a therapeutic substance transdermally, which system comprises:
a sheet of interconnected bandage elements defined by a plurality of separation lines, each bandage element comprising a sandwich-type laminate, the laminate including an impermeable backing lamina, the laminate further including a contact adhesive lamina connected with the backing lamina and being for adhering the bandage element to a user's skin, each bandage element further including a predetermined amount of a releasable form of the therapeutic substance communicatable by way of the adhesive lamina to the skin upon adherence of the bandage element to the skin, whereby a user may divide said sheet along the separation lines to obtain a desired number of bandage elements to be applied to the skin.

2. The system of claim 1 in which the adhesive lamina of each bandage element comprises a mixture of adhesive and of the therapeutic substance.

3. The system of claim 1 in which each bandage element includes a reservoir of the therapeutic substance contained between the backing lamina and the adhesive lamina.

4. The system of claim 1 in which the therapeutic substance is applied uniformly over the surface of each bandage element.

5. The system of claim 1 in which the therapeutic substance is applied uniformly over said sheet.

6. The system of claim 5 in which each of the bandage elements are of identical size.

7. The system of claim 1 in which the separation lines consist of perforation lines.

8. The system of claim 7 in which the perforation lines form a grid dividing said sheet into a rectangular array of bandage elements.

9. The system of claim 1 and which further includes a release liner covering the adhesive lamina of each of the bandage elements.
